# EUROPÄISCHE PATENTSCHRIFT

(11) **EP 0 384 951 B1**
(45) Veröffentlichungstag und Bekanntmachung des Hinweises auf die Patenterteilung: **08.06.1994**
(21) Anmeldenummer: 89113969.3
(22) Anmeldetag: 28.07.1989
(51) Int. Cl.: A61F 2/52

(54) **Brustprothese**
Mammary prosthesis
Prothèse mammaire

(30) Priorität: 27.02.1989 DE 8902304 U
(43) Veröffentlichungstag der Anmeldung: 05.09.1990
(73) Patentinhaber: Eberl, Tertulin, D-82377 Penzberg (DE); Anita-Spezialmiederfabrik Dr. Helbig Ges.m.b.H. & Co. KG, A-6330 Kufstein (AT)
(72) Erfinder: Weber-Unger, Georg, A-6330 Kufstein (AT); Eberl, Tertulin, D-8122 Penzberg (DE)
(74) Vertreter: Haug, Dietmar, Dipl.-Ing.

(56) Entgegenhaltungen:
- DE-B- 1 072 358
- DE-U- 8 902 304
- FR-A- 2 250 280
- GB-A- 2 059 775
- US-A- 2 752 602
- US-A- 3 278 947
- US-A- 4 195 639

## Beschreibung

Die Erfindung betrifft eine Brustprothese gemäß dem Oberbegriff des Anspruchs 1.

Die Form einer solchen Brustprothese ist aus dem US-Patent 4 195 639 bekannt. Die dort beschriebene Brustprothese wird senkrecht getragen, so daß der längere Ausläufer beim Tragen nach oben weist und den Bereich oberhalb der Mamma abdeckt, während ein seitlicher Ausläufer sich zur Mitte der Brust hin erstreckt und der andere zum Achselbereich hinweist. Eine solche Brustprothese kann aber nicht in einer um 90° gedrehten Position getragen werden, so daß das als obere Ausläufer gedachte Ende zur Achsel des Trägers zeigt und eines der als seitliche Ausläufer gedachten Enden unterhalb des Büstenhalters herausschaut, da dann beim Tragen der Prothese nicht mehr eine der menschlichen Brust nachgebildete Form entsteht. Die Abmessungen der flüssigkeitsgefüllten Hülle und die Lage der Brustspitze auf dieser Hülle sind so aufeinander abgestimmt, daß nur beim Tragen dieser Prothese in der senkrechten Position durch das Eigengewicht der Flüssigkeit eine der menschlichen Brust nachempfundene Form entsteht. Beim waagrechten Tragen einer solchen Prothese ist es auch nicht zu vermeiden, daß einer der seitlichen Ausläufer unten aus dem Büstenhalter hervorschaut. Dieser Ausläufer ist aber einerseits viel zu dick und groß, um zwischen Büstenhalter und Brust eingeklemmt zu werden, um das Verrutschen der Prothese zu verhindern. Andererseits ist ein solcher Ausläufer auch nicht geeignet unter den Büstenhalter geschoben zu werden, um die von der Prothese eingenommene Form positiv zu beeinflussen.

Die Aufgabe der Erfindung besteht darin, die gattungsgemäße symmetrische Brustprothese so auszubilden, daß sie sowohl einen stufenlosen Übergang zum Achselbereich der Trägerin bei gleichzeitigem vollständigen Ausgleich von entferntem Lymphdrüsengewebe schafft und außerdem einen stufenlosen Übergang zu der oberhalb der Mamma liegenden Körperpartie der Trägerin ergibt.

Die Aufgabe der Erfindung wird durch das Merkmal des kennzeichnenden Teils des Anspruchs 1 gelöst.

Die erfindungsgemäße Brustprothese vereinigt sämtliche Vorteile einer symmetrischen Brustprothese mit denen einer asymmetrischen Brustprothese, wobei der jeweils untere Ausläufer keinerlei Nachteile für die Trägerin bringt. Wie sich bei Versuchen überraschend gezeigt hat, wird der untere Ausläufer beim Tragen der Prothese durch die infolge des Eigengewichts der Prothese auf den unteren Ausläufer ausgeübte Stützkraft des Büstenhalters und die Weichheit des Prothesenmaterials nach oben geschoben, wobei sich der untere Ausläufer der Form des Büstenhalters anpaßt und die Vertiefung auf der Rückseite der Prothese entsprechend verformt wird, während der obere Ausläufer unverformt bleibt und seine Funktion, den oberen Brustansatz zu simulieren, voll behält.

Die erfindungsgemäße Prothese hat ferner den Vorteil, daß sie beim Tragen der Form der natürlichen Brust noch weiter angepaßt ist, da der nach oben geschobene untere Ausläufer die gleiche starke Rundung wie die der natürlichen Brust im Unterbrustbereich aufweist und wie die natürliche Brust im Unterbrustbereich eine Falte mit der unterhalb der Brust liegenden Körperpartie bildet.

Vorzugsweise hat jeder der weiteren Ausläufer eine Grundfläche, die kleiner als die Grundfläche des seitlichen Ausläufers ist. Hierdurch wird in Verbindung mit der erfindungsgemäßen Wahl der Längen der Ausläufer erreicht, daß bei Erhaltung der Symmetrie der obere Ausläufer nicht aus dem Büstenhalter herausragen kann und der seitliche Ausläufer ausreichend groß ist, um das wegoperierte Lymphdrüsengewebe auszugleichen und einen stufenlosen Übergang zum Achselbereich der Trägerin zu schaffen.

Ein wesentlicher Vorteil der erfindungsgemäßen Prothese besteht darin, daß sie wahlweise auf der rechten oder linken Brustseite getragen werden kann, wodurch sie auf Grund einer vereinfachten Herstellung und Lagerhaltung kostengünstiger wird.

Ein Ausführungsbeispiel der Erfindung ist in den Zeichnungen dargestellt und wird im folgenden näher beschrieben. Es zeigt
- Figur 1: eine Draufsicht auf die Vorderseite der erfindungsgemäßen Brustprothese,
- Figur 2: einen Schnitt durch die in Figur 1 gezeigte Brustprothese entlang der Linie II-II und
- Figur 3: einen Schnitt der in Figur 1 dargestellten Brustprothese entlang der Symmetrieebene SE.

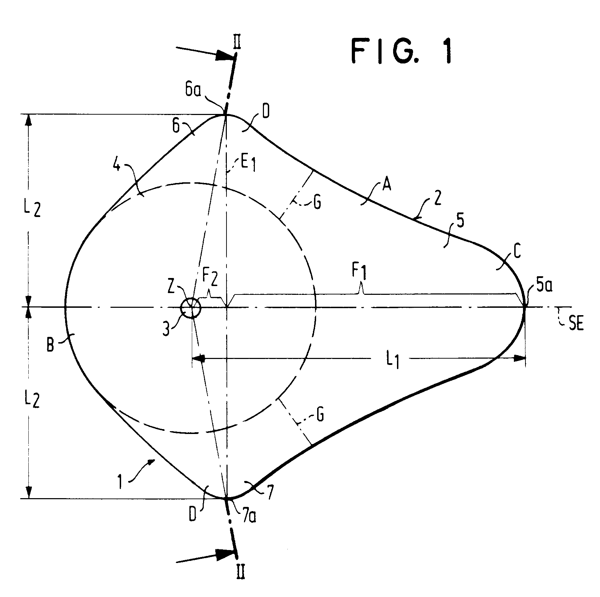

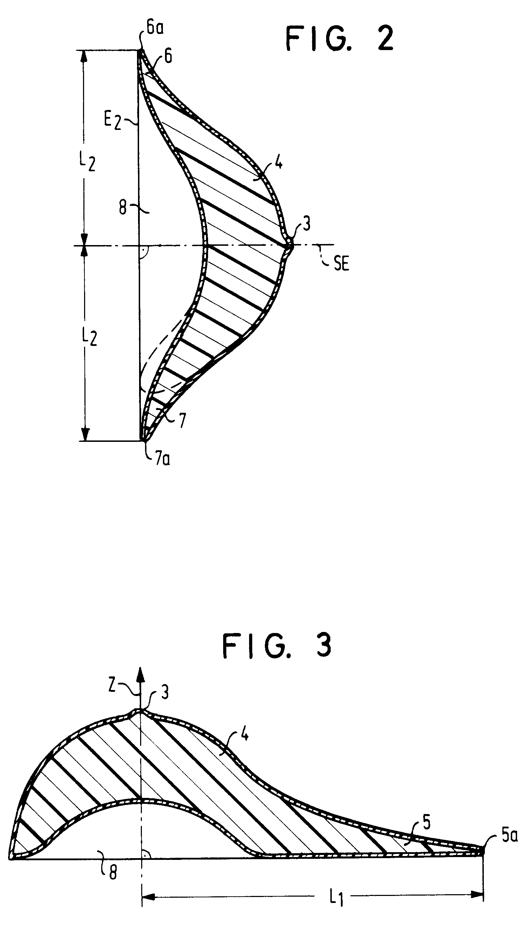

Eine erfindungsgemäße Brustprothese besteht aus einem weichelastischen schalenförmigen Kunststoffkörper 1, der im wesentlichen aus einer additionsvernetzten gelartigen Siliconkautschukmasse besteht, die von einer aus zwei Teilen bestehenden Kunststoffolie, z.B. einer Polyurethanfolie, umhüllt ist, wobei die beiden Folienteile längs des Prothesenrandes 2 miteinander verschweißt sind. Wie aus den Figuren 1 und 2 ersichtlich ist, hat der Kunststoffkörper 1 eine Form, die symmetrisch bezüglich einer durch seine Spitze 3 gehenden, senkrecht auf seiner Grundfläche A stehenden Symmetrieebene SE ist. Der Kunststoffkörper 1 besteht aus einem die Spitze 3 aufweisenden Hauptteil 4, der eine im wesentlichen kreisförmige Grundfläche B hat, deren Umkreis in Figur 1 insoweit gestrichelt eingezeichnet ist, als er innerhalb der gesamten Grundfläche A des Kunststoffkörpers 1 liegt. Die Spitze 3 des Kunststoffkörpers 1 ist auf der Vorderseite der Prothese angeordnet und hat die Form einer Brustwarze.

An den Hauptteil 4 schließt sich ein von der Symmetrieebene SE durchsetzter, seitlicher Ausläufer 5 an, der sich mit zunehmender Entfernung von dem Hauptteil 4 in seiner Grundfläche C zu seinem Ende 5a hin verjüngt und der, wie aus Figur 3 ersichtlich ist, senkrecht zu seiner Grundfläche C nach außen flacher wird.

Die Vorderseite des Kunststoffkörpers 1 hat eine der natürlichen Brust nachgebildete konvexe Form, während die Rückseite eine Vertiefung 8 aufweist, die etwas flacher als die Vorderseite der Prothese gewölbt ist. Der Kunststoffkörper 1 hat somit insgesamt gesehen eine Schalenform.

An den Hauptteil 4 schließen sich auf beiden Seiten der Symmetrieebene SE ein oberer und ein unterer Ausläufer 6 bzw. 7 an, die sich genau gegenüberliegen, d.h. die Enden 6a und 7a liegen in einer gemeinsamen ersten Ebene, welche die Symmetrieebene SE entlang einer nicht-dargestellten Linie rechtwinklig schneidet, die von der durch die Spitze 3 gehenden Z-Achse einen Abstand F₂ hat, der kleiner als der Abstand F₁ des Endes 5a des seitlichen Ausläufers 5 von dieser Linie ist. Der obere und der untere Ausläufer 6 bzw. 7, die auf Grund der Symmetrie des Kunststoffkörpers 1 zu der Symmetrieebene SE die gleiche Größe und Form haben, verjüngen sich in ihrer Grundfläche D zu ihren Enden 6a bzw. 7a hin und werden senkrecht zu ihrer Grundfläche D nach außen flacher. Die ungefähren Grenzen zwischen den Grundflächen D und der Grundfläche C sind in Figur 1 durch je eine strichpunktierte Linie G angedeutet.

Wie aus Figur 1 ersichtlich ist, haben das Ende 6a des oberen Ausläufers 6 und das Ende 7a des unteren Ausläufers 7 von der Symmetrieebene SE den gleichen Abstand L₂, der kleiner als der Abstand L₁ des Endes 5a des seitlichen Ausläufers 5 von der Z-Achse ist, die in der Symmetrieebene SE liegt, senkrecht auf der Grundfläche A steht und durch die Spitze 3 geht. Außerdem haben der obere und der untere Ausläufer 6 bzw. 7 jeweils eine kleinere Grundfläche D als der seitliche Ausläufer 5.

Wie aus Figur 2 ersichtlich ist, liegen die Enden 6a und 7a der Ausläufer 6 bzw. 7 in einer gemeinsamen zweiten Ebene, welche die Rückseite der Prothese begrenzt.

In Figur 2 ist gestrichelt die Form eingezeichnet, die der untere Ausläufer 7 und der sich anschließende Außenbereich des Hauptteils 4 annehmen, wenn die Brustprothese in einem Büstenhalter getragen wird. Durch die infolge des Eigengewichts der Prothese auf den unteren Ausläufer 7 ausgeübte Stützkraft des Büstenhalters wird der untere Ausläufer 7 nach oben geschoben, wobei er sich der Form des Büstenhalters anpaßt und sich die auf der Rückseite der Prothese vorhandene Vertiefung 8 entsprechend verformt. Durch die Verformung des unteren Ausläufers 7 beim Tragen der Prothese in einem Büstenhalter wird die Form der übrigen Teile der Prothese nicht beeinflußt. Wenn der untere Ausläufer 7 nach oben geschoben ist, hat er die gleiche starke Rundung wie die natürliche Brust im Unterbrustbereich und bildet wie die natürliche Brust eine Falte mit der sich unterhalb der Brust anschließenden Körperpartie.

Auf Grund der symmetrischen Ausbildung der erfindungsgemäßen Prothese in bezug auf die Symmetrieebene SE kann sie bei entsprechender Ausrichtung wahlweise auf der rechten oder linken Körperseite getragen werden.

Die Form und die Anordnung der Ausläufer ist nicht auf das Ausführungsbeispiel beschränkt, sondern kann innerhalb des durch die Ansprüche definierten Rahmens abgewandelt werden, solange die Symmetrie des Kunststoffkörpers in bezug auf die Symmetrieebene SE gewahrt bleibt.

## Patentansprüche

1. Brustprothese zum Tragen in einem Büstenhalter, bestehend aus einem weich-elastischen schalenförmigen, eine Vertiefung (8) auf der Rückseite der Prothese aufweisenden Kunststoffkörper (1), der symmetrisch bezüglich einer durch seine auf der Vorderseite der Prothese angeordneten Spitze (3) gehenden, senkrecht auf seiner Grundfläche (A) stehenden Symmetrieebene (SE) ist, einen die Spitze (3) aufweisenden Hauptteil (4) besitzt, einen an den Hauptteil (4) sich anschließenden, von der Symmetrieebene (SE) durchsetzten seitlichen Ausläufer (5), der sich zu seinem in der Symmetrieebene (SE) liegenden Ende (5a) verjüngt, und ein Paar weitere Ausläufer (6, 7) besitzt, die gegenüberliegend zu beiden Seiten der Symmetrieebene (SE) angeordnet sind, sich an den Hauptteil (1) anschließen und sich zu ihrem jeweiligen Ende (6a, 7a) hin verjüngen, wobei die Enden (6a, 7a) der weiteren Ausläufer (6, 7) einen Abstand (L2) von der Symmetrieebene (SE) aufweisen, der kleiner ist als der Abstand (L1) des Endes (5a) des seitlichen Ausläufers (5) zu einer z-Achse ist, die in der Symmetrieebene (SE) senkrecht zu der Grundfläche (A) durch die Spitze (3) verläuft, und wobei die Enden (6a, 7a) der weiteren Ausläufer (6, 7) in einer ersten gemeinsamen Ebene (E1) liegen, die die Symmetrieebene (SE) entlang einer Linie rechtwinklig schneidet dadurch gekennzeichnet, daß die Linie zwischen der z-Achse und dem Ende (5a) des seitlichen Ausläufers (5) verläuft.

2. Brustprothese nach Anspruch 1, dadurch gekennzeichnet, daß die Enden (6a, 7a) der weiteren Ausläufer (6, 7) in einer gemeinsamen zweiten Ebene (E2) liegen, welche die Rückseite der Prothese begrenzt.

## Claims

1. A breast prosthesis for wearing in a brassiere, comprising a soft-elastic dish-shaped plastic body (1) having a recess (8) on the rear side of the prosthesis, said body being symmetrical with respect to a symmetry plane (SE) which passes through its tip (3) disposed on the front side of the prosthesis and extends perpendicularly to its base area (A), having a main part (4) including the tip (3), a lateral extension (5) which adjoins the main part (4), is traversed by the symmetry plane (SE) and tapers towards its end (5a) lying in the symmetry plane (SE), and having a pair of further extensions (6, 7) which are arranged oppositely on both sides of the symmetry plane (SE), adjoin the main part (1) and taper towards their respective ends (6a, 7a), said ends (6a, 7a) of the further extensions (6, 7) having a distance (L2) from the symmetry plane (SE) which is smaller than the distance (L1) between the end (5a) of the lateral extension (5) and a Z-axis passing in the symmetry plane (SE) perpendicularly to the base area (A) through the tip, and the ends (6a, 7a) of the further extensions (6, 7) lying in a first common plane (E1) which intersects the symmetry plane (SE) at rights angles along a line, characterized in that the line extends between the Z-axis and the end (5a) of the lateral extension (5).

2. The breast prosthesis according to claim 1, characterized in that the ends (6a, 7a) of the further extensions (6, 7) lie in a common second plane (E2) which confines the rear side of the prosthesis.

## Revendications

1. Prothèse de poitrine à porter dans un soutien-gorge, constituée d'un corps (1) en matière plastique élastique molle, en forme de coquille, présentant un creux (8) sur la face arrière de la prothèse, corps (1) qui, étant symétrique par rapport à un plan de symétrie (SE) perpendiculaire à sa surface de base (A) et passant par sa pointe (3) disposée sur la face avant de la prothèse, possède une partie principale (4) présentant la pointe (3), possède un prolongement latéral (5), raccordé à la partie principale (4) et traversé par le plan de symétrie (SE), ce prolongement s'amincissant vers son extrémité (5a) située dans le plan de symétrie (SE), et possède un couple d'autres prolongements (6, 7), qui sont disposés en face l'un de l'autre, des deux côtés du plan de symétrie (SE), qui se raccordent à la partie principale (4) et qui s'amincissent vers leurs extrémités respectives (6a, 7a), étant entendu que les extrémités (6a, 7a) des autres prolongements (6, 7) se trouvent à une distance (L2) du plan de symétrie, plus petite que la distance (L1) de l'extrémité (5a) du prolongement latéral (5) par rapport à un axe (Z) passant par la pointe (3) dans le plan de symétrie (SE) et perpendiculaire à la surface de base (A), et étant entendu que les extrémités (6a, 7a) des autres prolongements (6, 7) sont situées dans un premier plan commun (E1) qui coupe le plan de symétrie (SE) perpendiculairement suivant une ligne, caractérisée en ce que cette ligne passe entre l'axe (Z) et l'extrémité (5a) du prolongement latéral (5).

2. Prothèse de poitrine suivant la revendication 1, caractérisée en ce que les extrémités (6a, 7a) des autres prolongements (6, 7) se trouvent dans un deuxième plan commun (E2), qui délimite la face arrière de la prothèse.
